# EUROPEAN PATENT APPLICATION

(11) **EP 4 461 288 A1**
(43) Date of publication of application: **13.11.2024**
(21) Application number: 22919090.5
(22) Date of filing: 28.12.2022
(51) Int. Cl.: A61K 8/14, A61K 8/55, A61K 8/68, A61K 8/63, A61K 8/73, A61Q 19/00

(54) **CATIONIC LIPOSOME FOR ENHANCING SKIN ABSORPTION AND PREPARATION METHOD THEREFOR**

(30) Priority: 06.01.2022 KR 20220002164
(71) Applicant: Cosmax, Inc., Hwaseong-si, Gyeonggi-do 18622 (KR)
(72) Inventor: KIM, Su Ji, Seongnam-si, Gyeonggi-do 13486 (KR); LEE, Jun Bae, Seongnam-si, Gyeonggi-do 13486 (KR); PARK, Myeong Sam, Seongnam-si, Gyeonggi-do 13486 (KR)
(74) Representative: Lavoix
(86) International application number: PCT/KR2022/021511
(87) International publication number: WO 2023/132553

(57) **Abstract**

Provided are a method for preparing cationic liposomes by using a high-pressure emulsification device, cationic liposomes prepared by the method, and a cosmetic composition, a food composition and a pharmaceutical composition, comprising the cationic liposomes. According to the method for preparing cationic liposomes, toxic organic solvents are not employed and thus it is possible to produce liposomes safe for the skin at mass scale by using a high-pressure emulsification device. In addition, the cationic liposomes prepared by the above method have excellent formulation stability, and are positively charged, so that the skin absorption ability of active ingredients loaded therein may be enhanced. Therefore, the cationic liposomes can be used in cosmetics, foods, drugs, and the like.

## Description

### Technical Field

This application claims priority benefits from Korean Patent Application No. 1 0-2022-0002164, filed on January 6, 2022, the entire contents of which are fully incorporated herein by reference.

The present disclosure relates to cationic liposomes for enhancing skin absorption of active ingredients, a cosmetic composition including the same, and a method for preparing the same.

### Background Art

Because the skin barrier is composed of lipid molecules that fill the extracellular space between stratum corneum cells, it plays a very important role in maintaining skin homeostasis and protecting the skin from changes due to various external physical stimuli and hydration/dehydration. However, in delivering active ingredients into the skin, such skin barrier works somewhat disadvantageously. In particular, it is known that active ingredients are most commonly delivered into the skin through the intercellular pathway, but it is more difficult for hydrophilic active ingredients to pass through this pathway, which is composed of lipid molecules. Therefore, to overcome this, a biocompatible dermal delivery system is needed.

Liposomes are a representative skin carrier used to enhance skin absorption of active ingredients, and since liposomes are composed of phospholipids, which are *in vivo* substances, they have high biocompatibility and thus are widely used in cosmetics or pharmaceuticals. In particular, they have a feature of being capable of loading hydrophilic and lipophilic substances simultaneously, making it advantageous for delivering various effective ingredients to the skin. In addition, since the active ingredients may be stably loaded in a bi-layer or multi-layer structure, they may be applied to a variety of cosmetic formulations, from emulsified formulations to solubilized formulations with a transparent appearance. Liposomes may be prepared into elastic liposomes, polymer-coated liposomes, cationic liposomes, etc., according to components of the membrane. Double cationic liposomes are liposomes with a positive zeta potential, which makes them more accessible to the skin due to electrostatic attraction with the negatively charged skin surface, helping to enhance skin absorption of active ingredients.

For the raw material of liposomes, it is most important to evaluate the stability and effectiveness of the loaded active ingredients when applied to the actual formulation. The most basic method of preparing liposomes is the thin film hydration method, but there are limitations in stability and mass production. Recently, a preparation method using a high-pressure emulsification device (microfluidizer) to prepare liposomes has been reported. However, when prepared using a high-pressure emulsification device, it is difficult to prepare bi-layer or multi-layer particles, which are unique particle types of liposomes. Therefore, it is necessary to confirm the shape of the liposomes prepared in this way and to evaluate their efficacy to determine whether they actually help deliver the active ingredients to the skin when applied in the formulation.

### Disclosure

### Technical Problem

An aspect provides a method for preparing cationic liposomes using a high pressure emulsification device.

Another aspect provides cationic liposomes prepared by the method.

Another aspect provides a cosmetic composition including the cationic liposomes.

Another aspect provides a composition for topical skin application including the cationic liposomes.

Another aspect provides a pharmaceutical composition including the cationic liposomes.

Another aspect provides a food composition including the cationic liposomes.

### Technical Solution

An aspect provides a method for preparing cationic liposomes using a high pressure emulsification device.

The method includes preparing a first solution including phospholipid, ceramide, and cholesterol, stirring a mixture of the first solution and a second solution including a cation donor material, and preparing the cationic liposomes from the stirred mixture using a high pressure emulsification device.

Among conventional liposomes preparation methods, the thin film hydration method uses organic solvents and has the problem that mass scale production is difficult. In contrast, the method for preparing cationic liposomes according to the aspect uses a high pressure emulsification device, so the use of organic solvents is unnecessary and mass scale production is also easy.

The method includes preparing a first solution including phospholipid, ceramide, and cholesterol.

The first solution may be prepared by dissolving by heating the components included in the first solution. The temperature during the dissolving by heating may be 60 °C to 90 °C, 60 °C to 85 °C, 60 °C to 80 °C, 65 °C to 90 °C, 65 °C to 85 °C, 65 °C to 80 °C, 70 °C to 90 °C, 70 °C to 85 °C, or 70 °C to 80 °C.

The phospholipid is a type of lipid including a polar phosphate and a non-polar fatty acid, and is a major component of biological membrane. The phospholipid may be a natural phospholipid or a synthetic phospholipid. The phospholipid may be, but is not limited to, lecithin, hydrogenated lecithin, phosphatidylcholine (PC), phosphatidylglycerol (PG), phosphatidylethanolamine (PE), phosphatidylserine (PS), phosphatidic acid (PA), phosphatidylinositol (PI), egg phosphatidylcholine (EPC), egg phosphatidylglycerol (EPG), egg phosphatidylethanolamine (EPE), egg phosphatidylserine (EPS), egg phosphatidic acid (EPA), egg phosphatidylinositol (EPI), soybean phosphatidylcholine (SPC), soybean phosphatidylglycerol (SPG), soybean phosphatidylethanolamine (SPE), soybean phosphatidylserine (SPS), soybean phosphatidic acid (SPA), soybean phosphatidylinositol (SPI), dipalmitoyl phosphatidylcholine (DPPC), 1,2-dioleoyl-sn-glycero-3-phosphatidylcholine (DOPC), dimyristoyl phosphatidylcholine (DMPC), dipalmitoyl phosphatidylglycerol (DPPG), diolelephosphatidylglycerol (DOPG), dimyristoyl phosphatidylglycerol (DMPG), hexadecylphosphocholine (HEPC), hydrogenated soybean phosphatidylcholine (HSPC), distearoyl phosphatidylcholine (DSPC), distearoyl phosphatidylglycerol (DSPG), dioleylphosphatidylethanolamine (DOPE), palmitoyl stearoyl phosphatidylcholine (PSPC), palmitoyl stearoyl phosphatidylglycerol (PSPG), monooleoyl phosphatidylethanolamine (MOPE), 1-palmitoyl-2-oleoyl-sn-glycero-3-phosphatidylcholine (POPC), polyethyleneglycol distearoylphosphatidylethanolamine (PEG-DSPE), dipalmitoyl phosphatidylserine (DPPS), 1,2-dioleoyl-sn-glycero-3-phosphatidylserine (DOPS), dimyristoyl phosphatidylserine (DMPS), distearoyl phosphatidylserine (DSPS), dipalmitoyl phosphatidic acid (DPPA), 1,2-dioleoyl-sn-glycero-3-phosphatidic acid (DOPA), dimyristoyl phosphatidic acid (DMPA), distearoylphosphatidic acid (DSPA), dipalmitoyl phosphatidylinositol (DPPI), 1,2-dioleoyl-sn-glycero-3-phosphatidylinositol (DOPI), dimyristoyl phosphatidylinositol (DMPI), distearoyl phosphatidylinositol (DSPI), or a combination thereof. The phospholipid may be hydrogenated lecithin.

The phospholipid may be included in an amount of 0.1 wt% to 5.0 wt%, 0.1 wt% to 4.0 wt%, 0.1 wt% to 3.0 wt%, 0.1 wt% to 2.0 wt%, 0.1 wt% to 1.0 wt%, 0.1 wt% to 0.7 wt% , 0.3 wt% to 5.0 wt%, 0.3 wt% to 4.0 wt%, 0.3 wt% to 3.0 wt%, 0.3 wt% to 2.0 wt%, 0.3 wt% to 1.0 wt%, 0.3 wt% to 0.7 wt%, 0.4 wt% to 2.0 wt%, 0.4 wt% to 1.0 wt%, 0.4 wt% to 0.7 wt%, or 0.4 wt% to 0.6 wt%, based on the total weight of the cationic liposome.

The ceramide may be, but is not limited to, ceramide NS, ceramide AS, ceramide EOS, ceramide NDS, ceramide ADS, ceramide EODS, ceramide NP, ceramide AP, ceramide EOP, ceramide NH, ceramide AH, ceramide EOH, or a combination thereof.

The ceramide may be included in an amount of 0.001 wt% to 0.5 wt%, 0.001 wt% to 0.1 wt%, 0.005 wt% to 0.5 wt%, 0.005 wt% to 0.1 wt%, 0.01 wt% to 0.5 wt%, 0.01 wt% to 0.1 wt%, or 0.01 wt% to 0.05 wt%, based on the total weight of the cationic liposome. The content of the ceramide may be selected by a person skilled in the art within an appropriate range to increase similarity to biological skin.

The weight ratio of the phospholipid and cholesterol may be 5:1 to 1:1, 4.5:1 to 1.5:1, 4:1 to 1.5:1,3.5:1 to 1.5:1, 3:1 to 1.5:1, 2.5:1 to 1.5:1,4.5:1 to 2:1,4:1 to 2:1, 3.5:1 to 2:1, 3:1 to 2:1, 2.5:1 to 2:2, or 2:1. If the cholesterol content is too small compared to the phospholipid content (e.g., the weight ratio of phospholipid and cholesterol is 5:1) or is equal to or greater than the phospholipid content (e.g., the weight ratio of phospholipid and cholesterol is 1:1), the phases are separated or suspension phenomenon may occur. Accordingly, the weight ratio of the phospholipid and cholesterol may be less than 5:1 to greater than 1:1. By using the above weight ratio of phospholipid and cholesterol, it is possible to obtain bi-layered or multi-layered particles, which are the unique particle shapes of liposomes, even if the liposomes preparation method using a high pressure emulsification device is used. In an example, when the weight ratio of phospholipid and cholesterol was 2:1, it was confirmed that the particle size and zeta potential did not change at room temperature for more than 5 weeks, indicating stability.

The cholesterol may be included in an amount of 0.1 wt% to 5.0 wt%, 0.1 wt% to 4.0 wt%, 0.1 wt% to 3.0 wt%, 0.1 wt% to 2.0 wt%, 0.1 wt% to 1.0 wt%, 0.1 wt% to 0.5 wt%, 0.1 wt% to 0.4 wt%, 0.1 wt% to 0.3 wt%, 0.2 wt% to 5.0 wt%, 0.2 wt% to 4.0 wt%, 0.2 wt% to 3.0 wt%, 0.2 wt% to 2.0 wt%, 0.2 wt% to 1.0 wt%, 0.2 wt% to 0.5 wt%, 0.2 wt% to 0.4 wt%, or 0.2 wt% to 0.3 wt%, based on the total weight of the cationic liposome. If the cholesterol content is outside the above range, the membrane stability of the liposome may decrease.

The first solution may include a solvent capable of dissolving phospholipid, cholesterol, and ceramide. The first solution may further include polyol. The first solution may further include dipropylene glycol. The dipropylene glycol may be used to dissolve lipid or oily substances. The dipropylene glycol may be included in an amount of 5 wt% to 20 wt%, 5 wt% to 15 wt%, 5 wt% to 12 wt%, 8 wt% to 20 wt%, 8 wt% to 15 wt%, 8 wt% to 12 wt, or 10 wt%, based on the total weight of the cationic liposomes, but is not limited thereto.

The first solution may further include additional ingredients that do not substantially affect the physical properties of the cationic liposomes. The content of the additional ingredients may be appropriately selected by a person skilled in the art depending on the purpose within a range that does not substantially affect the physical properties of the cationic liposomes.

The first solution may be composed of hydrogenated lecithin, cholesterol, ceramide, and dipropylene glycol, but is not limited thereto.

The method includes stirring a mixture of the first solution and a second solution including a cation donor material.

The second solution may include a cation donor material so that the finally prepared liposomes are positively charged (+). The cation donor material may be one or more selected from the group consisting of Laurdimonium Hydroxypropyl Hydrolyzed Wheat Protein, Guar Hydroxypropyltrimonium Chloride, Polyquaternium-7, and Polyquaternium. Polyquaternium-10, Polyquaternium-11, Polyquaternium-22, Polyquaternium-51, Polyquaternium-67, Polyquaternium-80, Cocamidopropylamine Oxide, Stearamidopropyl Dimethylamine, Behentrimonium Chloride, and chitosan, but it is not limited thereto. The cation donor material may be a cationic polysaccharide. The cationic polysaccharide may be a natural polysaccharide or a synthetic polysaccharide. The cation donor material may be chitosan.

The cation donor material may be included in an amount of more than 0.01 wt%, more than 0.01 wt% to 5.0 wt%, more than 0.01 wt% to 4.0 wt%, more than 0.01 wt% to 3.0 wt%, more than 0.01 wt% to 2.0 wt%, 0.05 wt% or more, 0.05 wt% to 5.0 wt%, 0.05 wt% to 4.0 wt%, 0.05 wt% to 3.0 wt%, 0.05 wt% to 2.0 wt%, 0.05 wt% to 1.5 wt%, 0.05 wt% to 1.0 wt%, 0.05 wt% to 0.5 wt%, 0.05 wt% to 0.2 wt%, or 0.05 wt% to 0.1 wt%, based on the total weight of the cationic liposome. When the cation donor material is included in an amount of 0.01 wt% or less based on the total weight of the cationic liposomes, the zeta potential of the liposomes may be negative (-), and anionic liposomes may be obtained accordingly. Therefore, the cation donor material is preferably included in an amount of more than 0.01 wt% based on the total weight of the cationic liposomes. When the cation donor material is included in an amount of 0.05 wt% or more based on the total weight of the cationic liposomes, cationic liposomes with a zeta potential of 20 mV or more may be obtained.

The second solution may include a solvent capable of dissolving the cation donor material. The second solution may include a hydrophilic solvent. For example, the hydrophilic solvent may be water (for example, distilled water), but is not limited thereto.

The second solution may further include butylene glycol. By including the butylene glycol, the solubility of the ingredients to be carried in the liposome may be improved and/or skin moisturizing ability thereof may be improved. The butylene glycol may be included in an amount of 5 wt% to 20 wt%, 5 wt% to 15 wt%, 5 wt% to 12 wt%, 8 wt% to 20 wt%, 8 wt% to 15 wt%, 8 wt% to 12 wt%, or 10 wt%, but is not limited thereto.

The second solution may further include a hydrophilic active ingredient. The hydrophilic active ingredient may be a skin functional active ingredient. The skin functionality may include, but is not limited to, skin whitening, moisturizing, skin barrier strengthening, antioxidant, anti-inflammatory, anti-aging, wrinkle improvement, skin soothing, and the like. The hydrophilic active ingredient may be used without limitation as long as it is known. For example, the hydrophilic active ingredient may be niacinamide.

The content of the hydrophilic active ingredient may be appropriately selected by a person skilled in the art within a range that does not substantially affect the physical properties of the cationic liposome, considering the type and purpose of the ingredient. Specifically, the hydrophilic active ingredient may be included in an amount of 0.001 wt% to 30 wt%, 0.001 wt% to 20 wt%, or 0.001 wt% to 10 wt% based on the total weight of the cationic liposomes. For example, when the hydrophilic active ingredient is niacinamide, the niacinamide is used in an amount of 0.001 wt% to 30 wt%, 0.001 wt% to 20 wt%, 0.001 wt% to 15 wt%, 0.01 wt% to 30 wt%, 0.01 wt% to 20 wt%, 0.01 wt% to 15 wt%, 0.1 wt% to 30 wt%, 0.1 wt% to 20 wt%, 0.1 wt% to 15 wt%, 1 wt% to 30 wt%, 1 wt% to 20 wt%, or 1 wt% to 15 wt%, based on the total weight of the cationic liposomes, but is not limited thereto.

The second solution may further include additional ingredients that do not substantially affect the physical properties of the cationic liposomes. The content of the additional ingredients may be appropriately selected by a person skilled in the art depending on the purpose within a range that does not substantially affect the physical properties of the cationic liposomes.

The stirring may be performed by slowly adding or mixing the first solution into the second solution.

The stirring may be performed at room temperature, for example, 15 °C to 35 °C, 15 °C to 30 °C, 20 °C to 35 °C, or 20 °C to 30 °C.

The stirring may be performed at 1,500 rpm to 3,500 rpm, 1,500 rpm to 3,000 rpm, 2,000 rpm to 3,500 rpm, or 2,000 rpm to 3,000 rpm. The stirring may be performed for 30 seconds to 30 minutes, 30 seconds to 20 minutes, 30 seconds to 10 minutes, 30 seconds to 5 minutes, 1 minute to 30 minutes, 1 minute to 20 minutes, 1 minute to 10 minutes, or 1 minute to 5 minutes.

The stirring may be performed according to a conventional method, for example, using a homomixer.

The method includes preparing cationic liposomes from the stirred mixture using a high pressure emulsification device.

In preparing the cationic liposomes, the stirred mixture may be subjected to high pressure emulsification using a high pressure emulsification device.

The type of the high pressure emulsification device is not limited as long as it is known, but for example, it may be a microfluidizer (MF) or a high pressure homogenizer.

The pressure conditions of the high pressure emulsifier may be 800 bar to 1500 bar, 800 bar to 1200 bar, 800 bar to 1100 bar, 800 bar to 1000 bar, 900 bar to 1500 bar, 900 bar to 1200 bar, 900 bar to 1100 bar, 900 bar to 1000 bar, 1000 bar to 1500 bar, 1000 bar to 1200 bar, 1000 bar to 1100 bar, or 1000 bar. If the pressure is too low, nanoparticles may not be formed properly. If the pressure is too high, excessive physical force may be applied and stability may decrease.

The high pressure emulsification device may be applied under the conditions of 2 cycles or more, 3 cycles or more, 2 cycles to 5 cycles, 2 cycles to 4 cycles, 3 cycles to 5 cycles, or 3 cycles.

When preparing cationic liposomes under the pressure and cycle conditions of the high pressure emulsification device, cationic nano liposomes with excellent formulation stability may be obtained.

When preparing the cationic liposomes under the pressure and cycle conditions of the high pressure emulsification device, bi-layered or multi-layered particles, which are unique particle shapes of liposomes, may be obtained.

Since the cationic liposomes are prepared using a high pressure emulsification device, toxic organic solvents are not employed and it is possible to produce liposomes safe for the skin at mass scale.

Another aspect provides cationic liposomes prepared by the method according to the above aspect.

The cationic liposomes prepared by the above method may be non-toxic because toxic organic solvents are not used during the preparation. In an embodiment, the cationic liposomes prepared by the above method and the cosmetic composition including the same were confirmed to be non-irritating and safe for the skin as a result of skin irritation index evaluation. Therefore, the cationic liposomes may be safely used in cosmetics, foods, drugs, and the like.

The particle size of the cationic liposomes may be 50 nm to 300 nm, 50 nm to 250 nm, 100 nm to 300 nm, 100 nm to 250 nm, 150 nm to 300 nm, 150 nm to 250 nm, 200 nm to 300 nm, or 200 nm to 250 nm. The average particle size of the cationic liposomes may be 200 nm to 250 nm. Therefore, the cationic liposomes may be nano liposomes. Since the cationic liposomes are nano-sized, they may have excellent skin penetration efficacy.

The zeta potential of the cationic liposomes may be 20 mV to 50 mV, 20 mV to 45 mV, 25 mV to 50 mV, or 25 mV to 40 mV. Since the cationic liposomes are positively charged (+), they may be effectively adsorbed to the negatively charged (-) skin surface using electrostatic attraction, thereby improving the skin absorption ability of the skin efficacy ingredients loaded in the liposomes.

Since the cationic liposomes maintain their particle size and zeta potential well even when stored at room temperature for more than 5 weeks, the formulation stability may be excellent.

The cationic liposomes may have a bi-layer or multi-layer structure. The cationic liposomes may be a mixture of liposomes with a bi-layer structure and liposomes with a multi-layer structure. In an embodiment, the cationic liposomes were confirmed to have a unique bi-layer or multi-layer liposome shape even though they were prepared using a high pressure emulsification device.

The cationic liposomes may be one in which skin functional active ingredients are loaded within the liposomes. The skin functional active ingredient may be a hydrophilic active ingredient. The skin functional active ingredient may be niacinamide, but is not limited thereto. In an embodiment, when niacinamide is delivered to the skin by being loaded in the cationic liposome, it was confirmed that the amount of change in skin brightness, the amount of change in melanin, and the amount of change in freckles were significantly increased, compared to the case where niacinamide is included in the composition without cationic liposomes and delivered to the skin. Therefore, the cationic liposomes may have improved skin absorption ability of the skin functional active ingredient loaded within the liposome.

Another aspect provides a composition including the cationic liposomes.

The composition may be a composition for delivering an active ingredient carried in the cationic liposomes. Specifically, the composition may be a composition for delivering an active ingredient loaded in the cationic liposomes to the skin.

The composition may include 0.001 wt% to 50 wt%, 0.001 wt% to 40 wt%, 0.001 wt% to 30 wt%, 0.001 wt% to 20 wt%, 0.01 wt% to 50 wt%, 0.01 wt% to 40 wt%, 0.01 wt% to 30 wt%, 0.01 wt% to 20 wt%, 0.1 wt% to 50 wt%, 0.1 wt% to 40 wt%, 0.1 wt% to 30 wt%, or 0.1 wt% to 20 wt% of the cationic liposomes, based on the total weight of the composition.

In an embodiment, the composition may be a cosmetic composition.

The cosmetic composition may further include, in addition to the active ingredients disclosed in this specification, ingredients commonly used in cosmetic compositions, functional additives, and the like, and it may include conventional auxiliaries such as antioxidants, stabilizers, solubilizers, surfactants, dispersants, thickeners, moisturizers, preservatives, vitamins, pigments, fragrances, etc., and cosmetically acceptable carriers.

The cosmetic composition may be prepared in any commonly prepared formulation. For example, the cosmetic composition may have a cosmetic formulation, such as toilet water, cream, essence, cleansing foam, cleansing water, pack, ampoule, body lotion, body oil, body gel, shampoo, rinse, hair conditioner, hair gel, foundation, lipstick, mascara, or a base makeup.

In an embodiment, the composition may be a composition for external application to the skin.

The composition for external application may be cream, gel, ointment, skin emulsifier, skin suspension, transdermal delivery patch, drug-containing bandage, lotion, or a combination thereof. The composition for external application may be appropriately mixed with ingredients commonly used in external skin preparations such as cosmetics and medicines, such as water-based ingredients, oil-based ingredients, powder ingredients, alcohols, moisturizers, thickeners, ultraviolet absorbers, whitening agents, preservatives, antioxidants, surfactants, fragrances, colorants, various skin nutrients, or a combination thereof, according to need.

In an embodiment, the composition may be a food composition. The food composition may be a health functional food composition.

The health functional food composition may be used alone or in combination with other foods or food ingredients, and may be used appropriately according to conventional methods. The mixing amount of the active ingredients may be appropriately determined depending on the purpose of use (prevention, health, or therapeutic treatment). There are no particular restrictions on the types of health functional foods. Among the types of health functional foods, beverage compositions may include various flavoring agents or natural carbohydrates as additional ingredients like ordinary beverages. The natural carbohydrates include monosaccharides such as glucose and fructose, disaccharides such as maltose and sucrose, polysaccharides such as dextrin and cyclodextrin, and sugar alcohols such as xylitol, sorbitol, and erythritol. As a sweetener, natural sweeteners such as thaumatin and stevia extract, or synthetic sweeteners such as saccharin and aspartame may be used. The health functional food composition may also include nutrients, vitamins, electrolytes, flavors, colorants, pectic acid and its salt, alginic acid and its salt, organic acids, protective colloidal thickeners, pH adjusters, stabilizers, preservatives, glycerin, alcohol, carbonating agents used for carbonated drinks, or a combination thereof. The health functional food composition may also include pulp for the preparation of natural fruit juice, fruit juice beverage, vegetable beverage, or a combination thereof.

In one embodiment, the composition may be a pharmaceutical composition.

The pharmaceutical composition may additionally include a pharmaceutically acceptable diluent or carrier. The diluent may be lactose, corn starch, soybean oil, microcrystalline cellulose, or mannitol, and the lubricant may be magnesium stearate, talc, or a combination thereof. The carrier may be an excipient, disintegrant, binder, lubricant, or a combination thereof. The excipient may be microcrystalline cellulose, lactose, low-substituted hydroxycellulose, or a combination thereof. The disintegrant may be calcium carboxymethylcellulose, sodium starch glycolate, calcium monohydrogen phosphate anhydride, or a combination thereof. The binder may be polyvinylpyrrolidone, low-substituted hydroxypropylcellulose, hydroxypropylcellulose, or a combination thereof. The lubricant may be magnesium stearate, silicon dioxide, talc, or a combination thereof.

The pharmaceutical composition may be formulated as an oral or parenteral dosage form. The oral dosage forms may be granules, powders, solutions, tablets, capsules, dry syrup, or a combination thereof. The parenteral dosage forms may be injectable.

Redundant content is omitted in consideration of the complexity of this specification, and terms not otherwise defined in this specification have meanings commonly used in the technical field to which the present disclosure pertains.

### Advantageous Effects

According to the method for preparing cationic liposomes according to an aspect, toxic organic solvents are not employed and thus it is possible to produce liposomes safe for the skin at mass scale, using a high pressure emulsification device.

In addition, the cationic liposomes prepared by the above method has excellent formulation stability and are positively charged, so that the skin absorption ability of the active ingredients loaded therein may be enhanced. Therefore, the cationic liposome may be used in cosmetics, foods, drugs, and the like.

### Description of Drawings

FIG. 1 shows the results of measuring the change in particle size (nm) of the cationic liposomes of Example 2-2 for 5 weeks after preparation.
FIG. 2 shows the results of measuring the change in zeta potential (mV) of the cationic liposomes of Example 2-2 for 5 weeks after preparation.
FIG. 3 shows the particle structure of the cationic liposomes of Example 2-2.
FIG. 4 is a graph showing skin brightness (L-value) after using the essence formulations of a Comparative Example and a Preparation Example.
FIG. 5 is a graph showing the amount of change in skin brightness after using the essence formulations of a Comparative Example and a Preparation Example.
FIG. 6 is a graph showing the melanin index (Ml) after using the essence formulations of a Comparative Example and a Preparation Example.
FIG. 7 is a graph showing the amount of change in skin melanin after using the essence formulations of a Comparative Example and a Preparation Example.
FIG. 8 shows the results of confirming skin blemishes (mm²) after using the essence formulations of a Comparative Example and a Preparation Example.
FIG. 9 is a graph showing the amount of change in skin blemishes after using the essence formulations of a Comparative Example and a Preparation Example.

### Best Mode

Hereinafter, the present disclosure will be described in more detail with reference to examples. However, these examples are only for illustrating the present disclosure, and the scope of the present disclosure is not limited to these examples.

### Example 1. Evaluation of physical properties of cationic liposomes according to chitosan content

Cationic liposomes were prepared by varying the chitosan content, and an experiment was conducted to measure their zeta potential.

First, cationic nanoliposomes were prepared with the ingredients and contents (wt%) in Table 1 below. The cationic liposomes of Examples 1-1 to 1-5 differ only in the content of chitosan.

The specific preparation method is as follows. Phase A consisting of lecithin and dipropylene glycol, was dissolved by heating to 70-80 °C. Phase A was slowly added to an aqueous phase B containing chitosan at room temperature and stirred with a homomixer at 2,000 to 3,000 rpm for 5 minutes. After primary stirring, liposomes were finally prepared by high-pressure emulsification treatment at 1,000 bar and 3 cycles.

**[Table 1]**

| | Ingredient | Content (wt%) | | | | |
|---|---|---|---|---|---|---|
| | | Example 1-1 | Example 1-2 | Examp le 1-3 | Example 1-4 | Example 1-5 |
| A | Hydrogenated Lecithin | 0.50 | 0.50 | 0.50 | 0.50 | 0.50 |
| | Dipropylene glycol | 10.00 | 10.00 | 10.00 | 10.00 | 10.00 |
| B | Distilled water | To 100 | To 100 | To 100 | To 100 | To 100 |
| | Butylene glycol | 10.00 | 10.00 | 10.00 | 10.00 | 10.00 |
| | Chitosan | - | 0.01 | 0.05 | 0.10 | 0.50 |
| | antiseptic | Appropria te amount | Appropri ate amount | Approp riate amoun t | Appropria te amount | Appropri ate amount |

Next, the zeta potential of the cationic liposomes according to the chitosan content was measured. A dynamic light scattering (DLS, SZ-100 HORIBA) device was used as a measurement device. The zeta potential measurement results of cationic liposomes according to chitosan content are shown in Table 2.

**[Table 2]**

| | Example 1-1 | Example 1-2 | Example 1-3 | Example 1-4 | Example 1-5 |
|---|---|---|---|---|---|
| zeta potential (zeta potential, mV) | -34.9 | -4.0 | 28.4 | 28.8 | 29.2 |

As shown in Table 2, the zeta potential of the cationic liposomes without chitosan (Example 1-1) was -34.9 mV. As the chitosan content increased, the zeta potential also increased, but when the chitosan content exceeded 0.1 wt%, the zeta potential no longer increased significantly. Accordingly, in the preparation of cationic liposomes, the content of chitosan was set to 0.05 wt% and further experiments were conducted.

### Example 2. Evaluation of physical properties of cationic liposomes according to cholesterol content

Cationic liposomes were prepared by keeping the chitosan content constant and varying the cholesterol content, and their stability was evaluated by measuring their particle size and zeta potential.

First, cationic liposomes were prepared using the same method as Example 1 with the ingredients and contents (wt%) in Table 3 below. The cationic liposomes of Examples 2-1 to 2-3 differ only in the content of cholesterol. Ceramide and cholesterol were used to improve similarity to the living body and skin safety. In the case of cholesterol, it is known to help improve the membrane stability of liposomes, so it was prepared with varying the cholesterol contents.

**[Table 3]**

| | Ingredient | Content (wt%) | | |
|---|---|---|---|---|
| | | Example 2-1 | Example 2-2 | Example 2-3 |
| **A** | Hydrogenated Lecithin | 0.50 | 0.50 | 0.50 |
| | **cholesterol** | **0.10** | **0.25** | **0.50** |
| | ceramide | 0.01 | 0.01 | 0.01 |
| | Dipropylene glycol | 10.00 | 10.00 | 10.00 |
| B | Distilled water | To 100 | To 100 | To 100 |
| | Butylene glycol | 10.00 | 10.00 | 10.00 |
| | Chitosan | 0.05 | 0.05 | 0.05 |
| | antiseptic | Appropri ate amount | Appropri ate amount | Appropri ate amount |

Next, stability was confirmed through visual evaluation immediately after preparation and after 3 days of storage at room temperature. As a result of visual evaluation, if the cholesterol content was too low or the same as lecithin (Examples 2-1 and 2-3), the phases were separated or a suspension phenomenon in which the color became cloudy occurred. On the other hand, in the cationic liposomes of Example 2-2, the phases were not separated and no suspension phenomenon occurred.

Therefore, the particle size and zeta potential of the cationic liposomes of Example 2-2 were measured immediately after preparation and weekly during 5 weeks of storage at room temperature using a dynamic light scattering device.

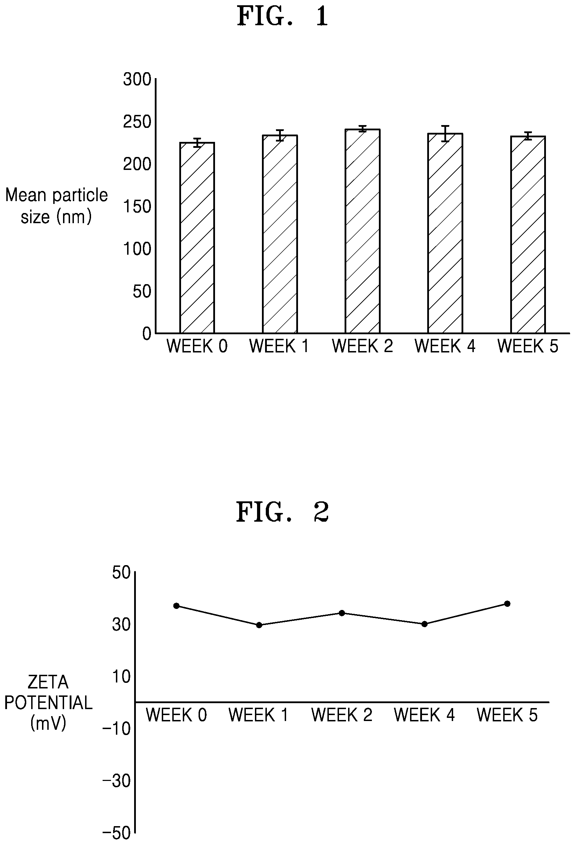

FIG. 1 shows the results of measuring the change in particle size (nm) of the cationic liposomes of Example 2-2 for 5 weeks after preparation.

FIG. 2 shows the results of measuring the change in zeta potential (mV) of the cationic liposomes of Example 2-2 for 5 weeks after preparation.

As shown in FIGS. 1 and 2, the particle size and zeta potential of the cationic liposomes of Example 2-2 in which the weight ratio of lecithin and cholesterol was 2:1 were stable for 5 weeks.

Meanwhile, the cationic liposomes of Examples 2-1 to 2-3 were prepared by varying only the high-pressure emulsification conditions in the preparation method of Example 1, and an experiment was conducted separately to measure their particle size. The three high-pressure emulsification conditions were set as follows: (i) 1000 bar, 1 cycle, (ii) 1000 bar, 3 cycles, (iii) 1000 bar, 5 cycles.

As a result of comparing particle sizes using a statistical program, there was a significant difference between 1 cycle and 3 cycles, but there was no significant difference between 3 cycles and 5 cycles. Therefore, it was confirmed that high pressure emulsification condition (ii) was most suitable.

### Experimental Example 1. Particle structure analysis of cationic liposomes

Particle structure analysis of the cationic liposome of Example 2-2 was performed.

Cryo-TEM (Cryogenic transmission electron microscopy) was used to analyze the structure of the prepared cationic liposome particles. Cryo-TEM is suitable for confirming the bi-layer or multi-layer structure of liposome particles because it maintains the sample at ultra-low temperature and allows observation of the original shape structure. The specific analysis method is as follows. 5 µL of the cationic liposomes of Example 2-2 was loaded onto a 200 mesh carbon lacey film Cu-grid and then was plunge-frozen by immersing it in ethane liquefied (approximately -170°C) with a Vitrobot. The prepared frozen sample was observed with Cryo-TEM (Tecnai F20, FEI) under an acceleration voltage of 200 kV.

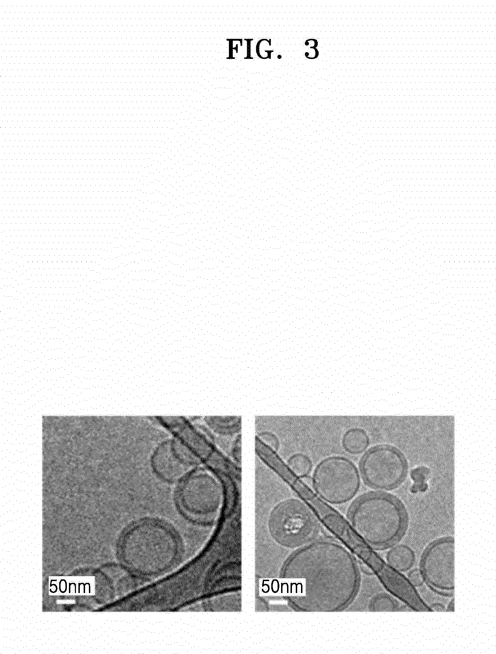

FIG. 3 shows the particle structure of the cationic liposomes of Example 2-2.

As shown in FIG. 3, the particle size of the cationic liposomes of Example 2-2 was about 100 to 300 nm. In addition, it was confirmed that bi-layer and multi-layer structures, which are characteristics of liposomes, were mixed and formed.

### Example 3. Preparation of cationic liposomes

10 wt% of niacinamide was loaded into the cationic liposomes of Example 2-2, which was confirmed to have the best stability through Examples 1 and 2. Specifically, cationic liposomes were prepared using the same method as Example 1 with the ingredients and contents (wt%) in Table 4 below.

**[Table 4]**

| | lingredient | Content (wt%) |
|---|---|---|
| | | Example 3 |
| A | Hydrogenated Lecithin | 0.50 |
| | cholesterol | 0.25 |
| | ceramide | 0.01 |
| | Dipropylene glycol | 10.00 |
| B | Distilled water | To 100 |
| | Butylene glycol | 10.00 |
| | Chitosan | 0.05 |
| | Niacinamide | 10.00 |
| | antiseptic | Appropriate amount |

### Preparation Example and Comparative Example. Preparation of cationic liposome-containing essence

As a preparation example, a cosmetic composition in the form of an essence formulation including the cationic liposome of Example 3 was prepared. Since the cationic liposomes of Example 3 includes 10 wt% of niacinamide, the cosmetic composition of the essence formulation including 20 wt% of the cationic liposome ultimately has a niacinamide content of about 2 wt%.

As a comparative example, an essence-type cosmetic composition including 2 wt% of niacinamide instead of cationic liposomes was prepared.

The ingredients and contents (wt%) of the essence formulation cosmetic compositions of the Preparation Example and Comparative Example are shown in Table 5 below.

**[Table 5]**

| | Ingredient | Content (wt%) | |
|---|---|---|---|
| | | Compar ative Example | Preparat ion Example |
| A | **Cationic liposomes of Example 3** | **-** | **20.00** |
| B | Dipropylene glycol | 5.00 | 3.00 |
| | coles-24 | 0.30 | - |
| | Hydrogenated Lecithin | - | 0.20 |
| C | Purified water | To 100 | To 100 |
| | Butylene glycol | 10.00 | 8.00 |
| | glycerin | 5.00 | 5.00 |
| | **Niacinamide** | **2.00** | **-** |
| | Pentylene glycol | 2.00 | 1.60 |
| | 1,2-hexanediol | 1.00 | 0.80 |
| | Sodium Phytate | 0.02 | - |
| | Triethanolamine | 0.08 | 0.08 |
| D | carbomer | Appropri ate amount | Appropri ate amount |
| | xanthan gum | Appropri ate amount | Appropri ate amount |
| | Ammonium Acryloyl Dimethyl Taurate/VPicopolymer | Appropri ate amount | Appropri ate amount |
| E | Fragrances | Appropri ate amount | Appropri ate amount |

### Experimental Example 2. Skin safety evaluation

Skin safety evaluation was performed on the cationic liposomes of Example 3 and the essence formulation of Preparation Example and Comparative Example.

Specifically, the degree of irritation of the cationic liposomes of Example 3 and the essence formulation of Preparation Example and Comparative Example was evaluated as follows in 20 adult men and women without skin disease. After applying 20 µL of the sample to the subjects' forearm, the test area was sealed and applied for 24 hours. The skin reaction was tested 30 minutes and 24 hours after removing the patch according to the terminology presented in the CTFA (The Cosmetic, Tioletry, and Fragrance Association) guidelines. The average of the subjects' Primary Irritation Index (PII) scores obtained according to the judgment criteria were evaluated as weak irritation if less than 1, mild irritation if less than 2, moderate irritation if less than 3.5, and strong irritation if more than 3.5. The results are shown in Table 6 below.

**[Table 6]**

| Experiment items | Cationic liposomes of Example 3 | Comparative Example | Preparation Example |
|---|---|---|---|
| Skin irritation index (PII) | No irritation | No irritation | No irritation |

As a result, as shown in Table 6, it was confirmed that both the cationic liposome of Example 3 and the essence formulation of Preparation Example and Comparative Example were non-irritating and could be safely used in products.

### Experimental Example 3. Whitening efficacy evaluation

The essence formulations of Preparation Example (with cationic liposome) and Comparative Example (without cationic liposome) each include 2 wt% of niacinamide, a whitening active ingredient. Therefore, the high whitening effect when using each formulation may be seen as a more efficient delivery of the active ingredients into the skin. To evaluate the whitening efficacy of the essence formulations of the Preparation Example and Comparative Example, three items of skin brightness, skin melanin, and skin blemishes were measured.

Specifically, 21 female subjects (36 to 58 years old, average 50.048 years old) were given each of the Comparative Example and Preparation Example products on half of their faces (half-test) twice a day (morning and evening) for 4 weeks. To measure skin brightness, melanin, and freckles, it was measured how much each value had changed after 2 and 4 weeks of use, compared to before use.

### (1) Skin brightness improvement effect

Skin brightness was photographed on the front of the face using Mark-Vu (PSIPLUS CO., LTD, KOREA), and the skin brightness value (L-value) of the test area was analyzed using the I-Max plus program on the captured images. As L-value increases, skin brightness improves.

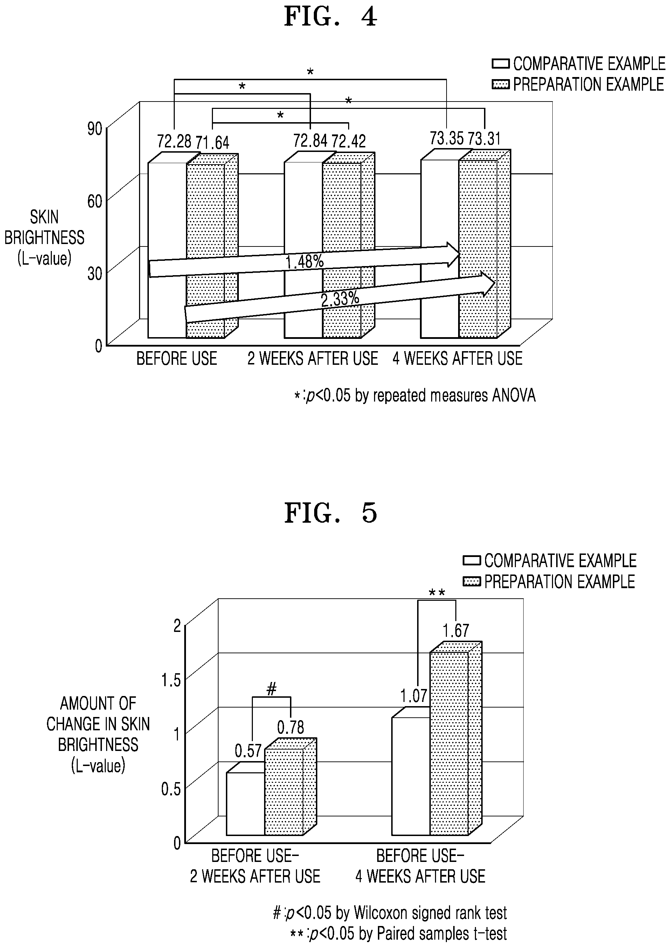

FIG. 4 is a graph showing skin brightness (L-value) after using the essence formulation of Comparative Example and Preparation Example. FIG. 5 is a graph showing the amount of change in skin brightness after using the essence formulation of Comparative Example and Preparation Example.

As shown in FIGS. 4 and 5, the Preparation Example showed a skin brightness improvement effect that was about 1.5 times better than the Comparative Example.

### (2) Skin melanin reduction effect

Skin melanin was measured using Mexameter MX18 (Courage Khazaka electroni, Germany), which indicates skin color. As the Melanin Index (MI) value decreases, it means that skin melanin improves.

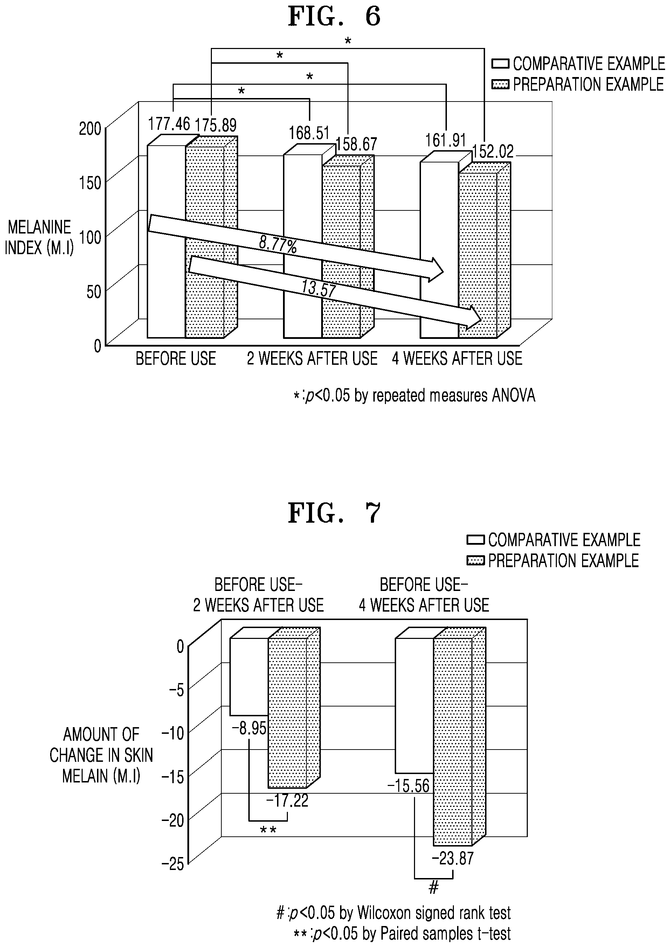

FIG. 6 is a graph showing the melanin index (MI) after using the essence formulation of Comparative Example and Preparation Example. FIG. 7 is a graph showing the amount of change in skin melanin after using the essence formulation of Comparative Example and Preparation Example.

As shown in FIGS. 6 and 7, the preparation example had a better effect in reducing skin melanin than the comparative example.

### (3) Efficacy in improving skin blemishes

Skin blemishes were measured using Antera 3D CS (Miravex, Ireland), and the affected area (mm²) in Melanin-Hyperconcentration analysis mode was evaluated. As freckles improve, the affected area (mm²) value decreases.

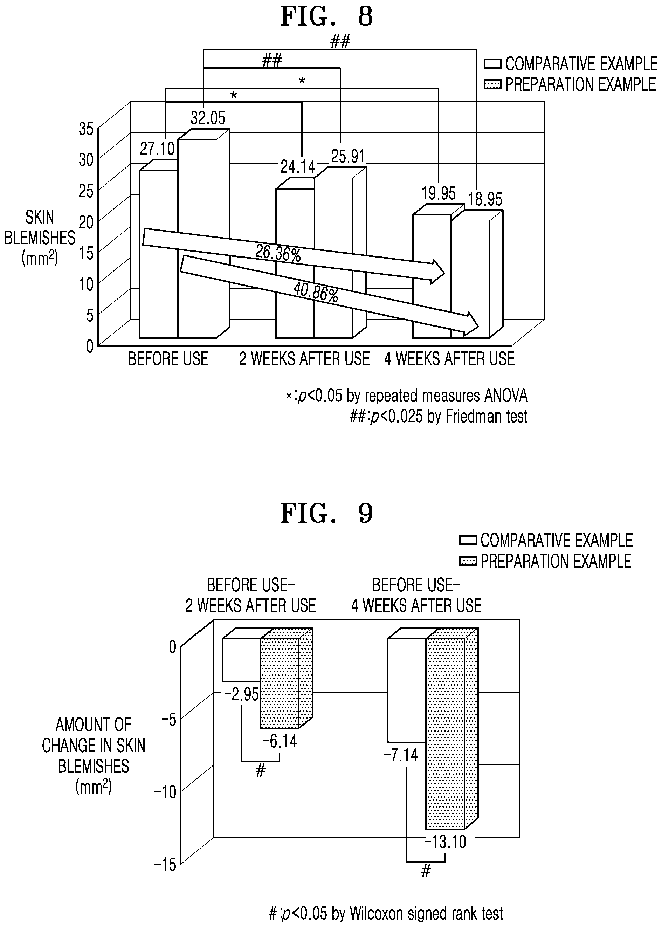

FIG. 8 shows the results of confirming skin blemishes (mm²) after using the essence formulations of Comparative Example and Preparation Example. FIG. 9 is a graph showing the amount of change in skin blemishes after using the essence formulation of Comparative Example and Preparation Example.

As shown in FIGS. 8 and 9, the Preparation Example showed a higher blemish improvement effect than Comparative Example.

Summarizing the results of Experimental Example 3, the amount of change in skin brightness, change in melanin, and change in freckles in Preparation Example showed significant differences compared to Comparative Example after 2 and 4 weeks of use. From this, it may be seen that the whitening effect was better when niacinamide was loaded in the cationic liposomes of Example 3. This can be attributed to the fact that the cationic liposomes better delivered active ingredients into the skin. Therefore, it may be seen that the cationic liposomes of Example 3 has excellent skin delivery efficacy of the loaded active ingredients.

## Claims

1. A method of preparing cationic liposomes, comprising:
preparing a first solution comprising phospholipids, ceramide, and cholesterol;
stirring a mixture of the first solution and a second solution comprising a cation donor material; and
preparing cationic liposomes from the stirred mixture by using a high-pressure emulsification device.

2. The method of claim 1, wherein the phospholipids are hydrogenated lecithin.

3. The method of claim 1, wherein the weight ratio of the phospholipid and cholesterol is 4.5:1 to 1.5:1.

4. The method of claim 1, wherein the cholesterol is comprised in an amount of 0.1 wt% to 5.0 wt%, based on the total weight of the cationic liposomes.

5. The method of claim 1, wherein the cation donor material is chitosan.

6. The method of claim 1, wherein the cation donor material is comprised in an amount of more than 0.01 wt% and up to 5.0 wt%, based on the total weight of the cationic liposomes.

7. The method of claim 1, wherein a pressure condition of the high-pressure emulsification device is 800 bar to 1500 bar.

8. Cationic liposomes prepared by the method according to any one of claims 1 to 7.

9. The cationic liposomes of claim 1, wherein a particle size of the cationic liposomes is 50 nm to 300 nm.

10. The cationic liposomes of claim 1, wherein a zeta potential of the cationic liposomes is 20 mV to 50 mV.

11. The cationic liposomes of claim 1, wherein the cationic liposomes have a bi-layer or multi-layer structure.

12. A cosmetic composition comprising the cationic liposomes according to claim 8.
